# EUROPÄISCHE PATENTSCHRIFT

(11) **EP 3 790 840 B1**
(45) Veröffentlichungstag und Bekanntmachung des Hinweises auf die Patenterteilung: **10.04.2024**
(21) Anmeldenummer: 19721282.2
(22) Anmeldetag: 02.05.2019
(51) Int. Cl.: B81C 1/00

(54) **VERFAHREN ZUM HERSTELLEN EINES BODENS EINER ANALYSEZELLE ZUM ANALYSIEREN EINES BIOCHEMISCHEN MATERIALS**
PROCESS FOR PRODUCING A BASE OF AN ANALYSIS CELL FOR ANALYSING A BIOCHEMICAL MATERIAL
PROCÉDÉ DE FABRICATION D'UN FOND D'UNE CELLULE D'ANALYSE PERMETTANT D'ANALYSER UN MATÉRIAU BIOCHIMIQUE

(30) Priorität: 08.05.2018 DE 102018207101
(43) Veröffentlichungstag der Anmeldung: 17.03.2021
(73) Patentinhaber: Robert Bosch GmbH, 70442 Stuttgart (DE)
(72) Erfinder: LAERMER, Franz, 71263 Weil Der Stadt (DE)
(86) Internationale Anmeldenummer: PCT/EP2019/061257
(87) Internationale Veröffentlichungsnummer: WO 2019/215011

(56) Entgegenhaltungen:
- LIU ET AL: "A templating route to nanoporous chitosan materials", CARBOHYDRATE RESE, PERGAMON, GB, Bd. 340, Nr. 18, 30. Dezember 2005 (2005-12-30), Seiten 2816-2820, XP005188321, ISSN: 0008-6215, DOI: 10.1016/J.CARRES.2005.09.022
- GRÉGORY F. SCHNEIDER ET AL: "DNA Translocation through Graphene Nanopores", NANO LETTERS, Bd. 10, Nr. 8, 11. August 2010 (2010-08-11), Seiten 3163-3167, XP055156495, ISSN: 1530-6984, DOI: 10.1021/nl102069z
- RHEE ET AL: "Nanopore sequencing technology: nanopore preparations", TRENDS IN BIOTECHNOL, ELSEVIER PUBLICATIONS, CAMBRIDGE, GB, Bd. 25, Nr. 4, 17. März 2007 (2007-03-17), Seiten 174-181, XP005932534, ISSN: 0167-7799, DOI: 10.1016/J.TIBTECH.2007.02.008

## Beschreibung

### Stand der Technik

Die Erfindung geht aus von einem Verfahren nach Gattung des unabhängigen Anspruchs.

Bekannt ist die Herstellung von Schichten durch Atomlagenabscheidung, auch atomic layer deposition oder kurz ALD genannt, und sogenannter selbstorganisierender Monoschichten aus Vorläufermolekülen, auch selfassembled monolayers oder kurz SAM genannt. Dabei docken die Vorläufermoleküle, auch Präkursoren oder Präkursormoleküle genannt, beispielsweise an Bindestellen auf einer Silizium- oder Glasoberfläche an, wobei abhängig von den Präkursoren auch eine Quervernetzung der Präkursormoleküle untereinander stattfinden kann. Diese Prozesse sind in der Regel selbstlimitierend, d. h., nach Abscheidung einer einzigen Monolage und Besetzung aller verfügbaren Bindungsplätze der Oberfläche findet keine weitere Abscheidung mit demselben Präkursor statt.

Bei der Atomlagenabscheidung wird durch zumindest einen weiteren Präkursor erreicht, dass auf einer ersten abgeschiedenen Monolage erneut Bindungsstellen gebildet werden, sodass vermöge des ersten Präkursors nachfolgend eine weitere Monolage abgeschieden werden kann. Diese Sequenz von mehreren Präkursoren kann so oft wiederholt werden, bis eine gewünschte Schichtdicke oder Anzahl von Monolagen abgeschieden wurde.

Bei selbstorganisierenden Monoschichten wird hingegen nur eine einzige Monolage auf der Oberfläche gebildet und meist auch in sich quervernetzt. Sobald die gesamte Oberfläche bedeckt ist, wobei auch alle verfügbaren Bindungsstellen der Oberfläche abgesättigt werden, werden keine Präkursormoleküle mehr auf der Oberfläche deponiert. Meistens wird dieses Verhalten dadurch erreicht, dass die Präkursormoleküle selektiv auf hydrophilen Oberflächen andocken, dabei selbst eine hydrophobe Oberfläche erzeugen und damit die weitere Abscheidung selbsttätig stoppen, weil weitere Präkursormoleküle auf hydrophoben Oberflächen nicht mehr andocken können.

Derartige Prozesse sind wohlbekannt und gut beherrscht. Eine ausgereifte Anlagentechnik steht sowohl für ALD-Schichten als auch für selbstorganisierende Monoschichten zur Verfügung. Oft kann sogar ein und dieselbe Anlage für beide Prozesstypen benutzt werden.

Selbstorganisierende Monoschichten können auch aus der Flüssigphase mithilfe geeigneter Lösungsmittel, beispielsweise Hexan oder Heptan, abgeschieden werden. Aufgrund der höheren Reinheit der Präkursorchemie und der besseren Qualität und Reproduzierbarkeit der abgeschiedenen Schichten hat sich jedoch die Gasphasenabscheidung durchgesetzt. Dabei werden flüssig vorgelagerte Präkursormoleküle in die Gasphase überführt, beispielsweise durch Erhitzen der Präkursorflüssigkeit. In einer Reaktionskammer wird der gewünschte Partialdruck des Präkursors eingestellt und mit dem Substrat in Kontakt gebracht. Es sind Methoden mit oder ohne Trägergas (Inertgas oder Edelgas wie beispielsweise N₂ oder Ar) bekannt.

Es ist weiter bekannt, dass gewisse SAM-Beschichtungen auf Wafern in qualitativ hochwertige Graphenschichten umgewandelt werden können. Dazu wird die SAM-Beschichtung nach der Abscheidung auf der Substratoberfläche beispielsweise durch Bedampfung mit einer 300 nm dicken Metallschicht, etwa aus Nickel oder Kupfer, überzogen und anschließend bei hoher Temperatur von 800 bis 1000 °C in Graphen umgewandelt.

Aus den Dokumenten Liu et al.: "A templating raute to nanoporous chitosan materials", CARBOHYDRATE RESE, PERGAMON, GB, Bd. 340, Nr. 18, 30. Dezember 2005 (2005-12-30), Seiten 2816-2820, XP005188321, ISSN: 0008-6215, DOI: 10.1016/J.CARRES.2005.09.022 und Schneider et al.: "DNA Translocation through Graphene Nanopores", NANO LETTERS, Bd. 10, Nr. 8, 11. August 201 O (2010-08-11), Seiten 3163-3167, XP055156495, ISSN: 1530-6984, DOI: 10.1021/nl102069z sind Verfahren zur Herstellung von Membranen mit Nanoporen bekannt. Eines dieses Verfahren umfasst die folgenden Schritte: Aufbringen kohlenstoffreicher Vorläufermoleküle und kohlenstoffarmer Vorläufermoleküle in einem definierten Mischungsverhältnis auf einem Substrat, um eine Vorläuferschicht zu bilden, wobei die kohlenstoffarmen Vorläufermoleküle eine definierte Größe und eine hydrophobe Terminierung aufweisen, und Nachbehandeln der Vorläuferschicht, um eine Schicht mit zumindest einer Pore mit einer von der definierten Größe abhängigen Porengröße und einer vom definierten Mischungsverhältnis abhängigen Porenanzahl herzustellen.

### Offenbarung der Erfindung

Vor diesem Hintergrund wird mit dem hier vorgestellten Ansatz ein Verfahren zum Herstellen eines Bodens einer Analysezelle zum Analysieren eines biochemischen Materials gemäß dem Hauptanspruch vorgestellt. Durch die in den abhängigen Ansprüchen aufgeführten Maßnahmen sind vorteilhafte Weiterbildungen und Verbesserungen des im unabhängigen Anspruch angegebenen Verfahrens möglich.

Die Sequenzierung eines biochemischen Materials, insbesondere von DNA-Abschnitten, kann beispielsweise mithilfe einer Membran aus Graphen oder einer nanometerdicken ALD-Schicht, die am Boden einer Arrayzelle für eine quantitative Polymerasekettenreaktion, kurz qPCR genannt, jeweils eine oder wenige Nanoporen aufweist, durchgeführt werden. Die Vorbereitung der Sequenzierung erfolgt dabei beispielsweise durch eine LATE-qPCR (LATE = linear after the exponential) in den Arrayzellen mit stark asymmetrischer Anzahl von für die jeweilige Arrayzelle charakteristischen Vorwärts- und Rückwärtsprimern spezifisch für jeweils einen charakteristischen DNA-Abschnitt, kombiniert mit einem für die jeweilige Arrayzelle charakteristischen Stoppnukleotid vom Typ A*, T*, C* oder G* nach Art einer Sanger-Sequenziermethode. Beim Durchtritt von vor allem während der linearen Phase der Polymerasekettenreaktion generierten sequenzspezifischen DNA-Fragmenten, die mit dem jeweils für die Arrayzelle charakteristischen Stoppnukleotid enden, durch die Nanoporen am Boden jeder qPCR-Arrayzelle kann eine Veränderung eines elektrischen Stromflusses gemessen werden und damit die Durchtrittszeit des jeweiligen DNA-Fragments durch die Nanopore bestimmt werden. Somit kann die jeweilige Länge der durchtretenden Fragmente ermittelt werden. Die Summe aller Fragmentlängen für jeweils vier Arrayzellen mit denselben Primern und den Stoppnukleotiden vom Typ A*, T*, C* oder G* (jeweils ein Typ in jeder der vier Arrayzellen) ergibt die gesamte DNA-Sequenz des entsprechenden durch die Primer festgelegten DNA-Abschnitts.

Der hier vorgestellte Ansatz ermöglicht nun, solche Nanoporen kostengünstig, einfach und schnell in Graphen- oder ALD-Schichten herzustellen, indem kohlenstoffarme Vorläufermoleküle mit hydrophober Terminierung in einem bestimmten Mischungsverhältnis mit kohlenstoffreichen Vorläufermolekülen auf einem Substrat angereichert werden, wobei die kohlenstoffarmen Vorläufermoleküle dazu beitragen, Lücken als Nanoporen in der sich ergebenden Schicht auszubilden. Ein entsprechendes Verfahren zur Erzeugung von Nanoporen ist universell sowohl für ALD-Schichten als auch für die Erzeugung von Graphen auf Basis selbstorganisierender Monoschichten geeignet und für beides einsetzbar. Die kohlenstoffreichen Vorläufermoleküle und die kohlenstoffarmen Vorläufermoleküle können sich in der Anzahl der von den Vorläufermolekülen umfassten Kohlenstoffatome unterscheiden, wobei die kohlenstoffreichen Vorläufermoleküle eine größere Anzahl von Kohlenstoffatomen aufweisen können.

Ein kohlenstoffarmes Vorläufermolekül, auch Precursor genannt, steuert für eine nachfolgende Umwandlungsreaktion in eine Graphen-artige Schicht wenig bis gar keinen Kohlenstoff bei. Ein kohlenstoffreicher Precursor steuert entsprechend viel Kohlenstoff bei.

Beispiele für kohlenstoffarme Vorläufermoleküle:
CI3-Si-CH3 (nur ein C-Atom pro Precursor)
Cl3-Si-NH2, Cl3-Si-H, Cl2-Si-H2, SiCl4,..... (gar kein C-Atom pro Precursor)

Beispiele für kohlenstoffreiche Vorläufermoleküle:
Cl3-Si-C6H5, Cl3-Si-(CnH2n+1), Cl2-Si-(CnH2n+1)-(CmH2m+1), .... (n, m können große Zahlen >10 sein)

Es wird ein Verfahren zum Herstellen eines Bodens einer Analysezelle zum Analysieren eines biochemischen Materials vorgestellt, wobei das Verfahren folgende Schritte umfasst:
Abscheiden kohlenstoffreicher Vorläufermoleküle undkohlenstoffarmer Vorläufermoleküle in einem definierten Mischungsverhältnis auf einem Substrat, um eine Vorläuferschicht zu bilden, wobei die kohlenstoffarmen Vorläufermoleküle eine definierte Größe und eine hydrophobe Terminierung aufweisen wobei die kohlenstoffreichen Vorläufermoleküle und/oder die kohlenstoffarmen Vorläufermoleküle mittels Atomlagenabscheidung und/ oder Gasphasenabscheidung und/oder Flüssigphasenabscheidung abgeschieden werden; und Nachbehandeln der Vorläuferschicht, um den Boden als Schicht mit zumindest einer Pore mit einer von der definierten Größe abhängigen Porengröße und einer vom definierten Mischungsverhältnis abhängigen Porenanzahl herzustellen.

Unter einer Analysezelle kann im Allgemeinen eine Kaverne zum Vorlagern des biochemischen Materials, etwa zum Durchführen einer Polymerasekettenreaktion, verstanden werden. Beispielsweise kann die Analysezelle als qPCR-Arrayzelle ausgebildet sein. Unter einem Boden kann ein Wandabschnitt mit zumindest einer Öffnung verstanden werden, durch die das biochemische Material aus der Analysezelle austreten kann, etwa zur Bestimmung einer Länge einzelner Fragmente des biochemischen Materials oder zur Sequenzierung des biochemischen Materials. Bei dem biochemischen Material kann es sich beispielsweise um eine Abfolge von Nukleotiden, etwa DNA, oder Ähnliches handeln. Unter einem Vorläufermolekül, auch Präkursormolekül genannt, kann ein Molekül eines schichtbildenden Ausgangsstoffes zum Abscheiden einer Schicht auf einem Substrat verstanden werden. Bei solchen Präkursoren kann es sich beispielsweise um organische Trichlorsilane, Trimethoxysilane oder Triethoxysilane, beispielsweise Octyltrichlorsilan, Decyltrichlorsilan, Octadecyltrichlorsilan, Phenyltrichlorsilan, Octyltrimethoxysilan, Decyltrimethoxysilan, Octadecyltrimethoxysilan, Phenyltrimethoxysilan, Octyltriethoxysilan, Decyltriethoxysilan, Octacecyltriethoxysilan oder Phenyltriethoxysilan, handeln. Besonders geeignet sind Trichlorsilane, da diese aufgrund ihrer hohen chemischen Reaktivität sehr schnell hydrolisieren und an Hydroxylgruppen hydrophiler Oberflächen binden und auch schnell quervernetzen. Es können jedoch auch Methoxy- oder Ethoxysilane eingesetzt werden. Für die Graphenerzeugung besonders geeignet sind Phenylsilane, insbesondere Trichlorphenylsilan, da diese Präkursormoleküle bereits aromatische Kohlenstoffringe enthalten, die der späteren Graphenstruktur entsprechen. Das Ausgangsmaterial ist im Fall von Phenyltrichlorsilan von seiner Struktur bereits ähnlich dem Zielmaterial, was die nachfolgende thermische Umwandlung der aromatischen Kohlenstoffringe zum Graphen fördert. Unter einem Substrat kann beispielsweise ein Silizium- oder Glassubstrat verstanden werden. Die Abscheidung der Vorläufermoleküle kann je nach Ausführungsform in einer Gas- oder Flüssigphase erfolgen. Unter einer Vorläuferschicht kann beispielsweise eine selbstorganisierende Monoschicht oder eine durch Atomlagenabscheidung erzeugte Schicht, auch ALD-Schicht genannt, verstanden werden. Unter einem kohlenstoffarmen Vorläufermolekül kann ein Molekül verstanden werden, das im Gegensatz zu einem kohlenstoffreichen Vorläufermolekül einen Mangel an Kohlenstoff aufweist. Beispielsweise können die kohlenstoffarmen Vorläufermoleküle kohlenstofffrei sein oder nur sehr wenige Kohlenstoffatome aufweisen. Die kohlenstoffarmen Vorläufermoleküle können beispielsweise auf einer Trichlorsilanchemie basieren und durch eine Stickstoff- oder Amidgruppe hydrophob terminiert sein. Unter einer Pore kann eine Nanopore mit einem Durchmesser im Nanometerbereich verstanden werden.

Unter Nachbehandeln kann beispielsweise ein chemisches, thermisches oder mechanisches Nachbehandeln der Vorläuferschicht verstanden werden. Beispielsweise kann dabei zumindest eines der kohlenstoffarmen Vorläufermoleküle eine die Pore repräsentierende Lücke in der Graphenschicht ausbilden. Alternativ können die kohlenstoffarmen Vorläufermoleküle beim Nachbehandeln zumindest teilweise aus der Vorläuferschicht entfernt werden, beispielsweise mittels Ätzen.

Gemäß einer Ausführungsform kann im Schritt des Nachbehandelns die Vorläuferschicht thermisch in eine Graphenschicht mit der Pore umgewandelt werden. Dadurch wird eine schnelle Herstellung des Bodens mit hoher Reinheit und hoher Wiederholgenauigkeit ermöglicht.

Gemäß einer weiteren Ausführungsform können im Schritt des Nachbehandelns die kohlenstoffarmen Vorläufermoleküle aus der Vorläuferschicht zumindest teilweise entfernt werden, um die Pore zu erzeugen. Auch durch diese Ausführungsform wird eine schnelle Herstellung des Bodens mit hoher Reinheit und hoher Wiederholgenauigkeit ermöglicht, insbesondere wenn die Vorläuferschicht mittels Atomlagenabscheidung gebildet wird.

Des Weiteren ist es vorteilhaft, wenn im Schritt des Abscheidens die kohlenstoffarmen Vorläufermoleküle in Form von Nanopartikeln abgeschieden werden. Unter Nanopartikeln können vorgefertigte Partikel mit einer Größe im Nanometerbereich verstanden werden, beispielsweise siliziumbasierte Nanopartikel, auch Silika-Beads genannt, oder ähnlich geeignete, kommerziell erhältliche Nanopartikel. Dadurch wird eine besonders kostengünstige Herstellung des Bodens ermöglicht.

Das Verfahren kann einen Schritt des Bildens von Molekülclustern aus zumindest zwei kohlenstoffarmen Vorläufermolekülen in einer Flüssigphase umfassen. Dabei können im Schritt des Abscheidens die Molekülcluster auf dem Substrat abgeschieden werden, um die Vorläuferschicht zu bilden. Dadurch kann ein Durchmesser der im Boden zu erzeugenden Pore mit verhältnismäßig geringem Aufwand sehr genau und kontrolliert eingestellt werden.

Dabei können die Molekülcluster im Schritt des Bildens durch Polykondensation und/oder Polymerisation gebildet werden. Dadurch können die Molekülcluster besonders einfach und schnell gebildet werden.

Gemäß einer weiteren Ausführungsform können im Schritt des Abscheidens die kohlenstoffreichen Vorläufermoleküle und die kohlenstoffarmen Vorläufermoleküle in einem Mischungsverhältnis abgeschieden werden, das so definiert ist, dass im Schritt des Nachbehandelns der Boden als Schicht mit maximal drei Poren hergestellt wird. Dadurch kann verhindert werden, dass der Boden mit einer zu großen Anzahl an Poren hergestellt wird.

Gemäß einer weiteren Ausführungsform kann im Schritt des Abscheidens eine mit den kohlenstoffarmen Vorläufermolekülen angereicherte selbstorganisierende Monoschicht als die Vorläuferschicht gebildet werden. Durch diese Ausführungsform kann sichergestellt werden, dass der Boden als möglichst defektarme und gleichmäßig strukturierte Schicht hergestellt wird.

Des Weiteren kann in einem Schritt des Vorbehandelns das Substrat vorbehandelt werden, um eine hydrophobe Oberfläche des Substrats zu erzeugen. In einem Schritt des Beaufschlagens kann die hydrophobe Oberfläche mit Wasserdampf beaufschlagt werden, um eine statistisch mit OH-Gruppen angereicherte Oberfläche zu erzeugen. Schließlich können im Schritt des Abscheidens die kohlenstoffarmen Vorläufermoleküle statistisch auf der statistisch mit OH-Gruppen angereicherten Oberfläche abgeschieden werden, um nachfolgend eine hydrophile Oberfläche mit hydrophoben Inseln aus den kohlenstoffarmen Vorläufermolekülen zu erzeugen. Dabei können die kohlenstoffreichen Vorläufermoleküle auf der nachfolgend hydrophil gemachten Oberfläche abgeschieden werden, um die Vorläuferschicht zu bilden. Dadurch kann der Boden mit besonders hoher Reinheit und Qualität hergestellt werden.

Es kann somit eine "hydrophobe Vorbehandlung der Oberfläche" durchgeführt werden. Dabei kann die Oberfläche ganzflächig hydrophob gemacht werden, z.B. unter Verwendung von Flusssäure. Anschließend kann die ganzflächig hydrophobe Oberfläche statistisch hydrophil gemacht werden. Darunter kann verstanden werden, dass die Oberfläche gerade nicht gleichmäßig hydrophil gemacht wird, sondern dass wenige hydrophile Inseln in statistischer Gleichverteilung über die Oberfläche durch Einwirkung von Wasserdampf erzeugt werden. Der statistischen Verteilung der durch Wasserdampf erzeugten hydrophilen Inseln besonders zuträglich ist dabei die Tatsache, dass diese Hydrophilisierung von Siiliziumoxidoberflächen nach einer Flusssäurebehandlung von statistisch verteilten Fluoratomen auf der Oberfläche gegenüber einer im allgemeinen dominierenden Wasserstoffterminierung startet, und sich dann langsam ausgehend von diesen "Keimen" über die Siliziumoxidoberfläche ausbreitet, bis der Hydrophilisierungsprozess bei Erreichung der gewünschten Größe der hydrophilen Inseln gestoppt wird. Die Anordnung der Inseln kann somit im Rahmen der Prozessparameter zufällig sein. Im Folgenden können diese statistisch gleichverteilten hydrophilen Inseln dann durch Beschichtung mit kohlenstoffarmen Precursor'n, welche dort fixiert werden, kohlenstoffarm gemacht werden. Anschließend kann der Rest der Oberfläche komplett hydrophil gemacht werden, beispielsweise durch Wasserdampfeinwirkung. Anschließend kann der Rest der Oberfläche kohlenstoffreich beschichtet werden vermöge kohlenstoffreicher Precursor.

Gemäß einer weiteren Ausführungsform können im Schritt des Abscheidens die kohlenstoffarmen Vorläufermoleküle bei einer Temperatur von 5 bis 30 °C abgeschieden werden. Zusätzlich oder alternativ können im Schritt des Abscheidens die kohlenstoffreichen Vorläufermoleküle bei einer Temperatur von größer 40 °C abgeschieden werden. Dadurch wird eine verhältnismäßig einfache temperaturgesteuerte Herstellung des Bodens ermöglicht.

Beispielsweise können im Schritt des Abscheidens die kohlenstoffreichen Vorläufermoleküle nach Abscheiden der kohlenstoffarmen Vorläufermoleküle nach Ablauf einer Vernetzungszeit, während der die abgeschiedenen kohlenstoffarmen Vorläufermoleküle bei einer Vernetzungstemperatur miteinander und/oder mit dem Substrat vernetzt werden, abgeschieden werden. Dadurch können die kohlenstoffreichen Vorläufermoleküle auf einer fest vernetzten Struktur abgeschieden werden.

Je nach Ausführungsform können im Schritt des Abscheidens die kohlenstoffreichen Vorläufermoleküle und/oder die kohlenstoffarmen Vorläufermoleküle mittels Atomlagenabscheidung, Gasphasenabscheidung, Flüssigphasenabscheidung oder mittels mehrerer der genannten Beschichtungsverfahren abgeschieden werden. Dadurch wird eine effiziente, kostengünstige Herstellung des Bodens ermöglicht.

Gemäß einem weiteren Ausführungsbeispiel können im Schritt des Abscheidens kohlenstoffarme Vorläufermoleküle abgeschieden werden, die durch zumindest eine Stickstoffgruppe und/oder zumindest eine Amidgruppe hydrophob terminiert sind. Dadurch wird eine einfache Synthese der kohlenstoffarmen Vorläufermoleküle ermöglicht.

Der hier vorgestellte Ansatz schafft schließlich eine Analysezelle zum Analysieren eines biochemischen Materials, mit einem Boden, der in einem Verfahren gemäß einer der vorstehenden Ausführungsformen als Schicht mit zumindest einer Pore hergestellt wurde.

Ausführungsbeispiele der Erfindung sind in den Zeichnungen dargestellt und in der nachfolgenden Beschreibung näher erläutert. Es zeigt:
- Fig. 1: eine schematische Darstellung einer Analysezelle welche erhaltbar gemäß einem Ausführungsbeispiel der Erfindung ist;
- Fig. 2: eine schematische Darstellung einer Struktur eines kohlenstoffarmen Vorläufermoleküls zur Verwendung in einem Verfahren gemäß einem Ausführungsbeispiel der Erfindung; und
- Fig. 3: ein Ablaufdiagramm eines Verfahrens gemäß einem Ausführungsbeispiel der Erfindung.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

Im Folgenden wird das Verfahren beispielhaft für die Erzeugung von Nanoporen in Graphen detailliert beschrieben. Es ist für den Fachmann jedoch nachvollziehbar, dass das Verfahren auch bei ALD-Prozessen funktioniert und ohne Einschränkung auf diese appliziert werden kann.

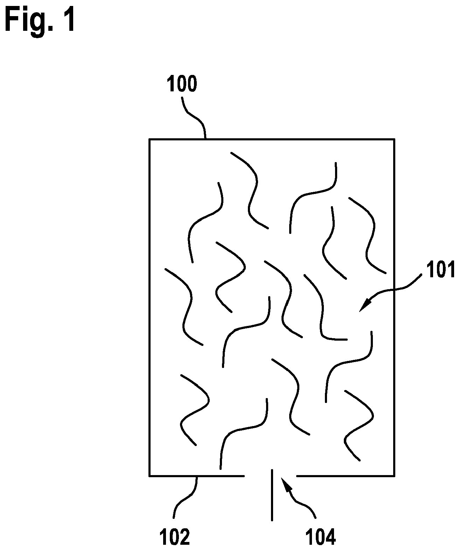

Fig. 1 zeigt eine schematische Darstellung einer Analysezelle 100 gemäß einem Ausführungsbeispiel. Die Analysezelle 100 zum Analysieren eines biochemischen Materials 101, beispielsweise eine Arrayzelle zur Durchführung einer quantitativen Polymerasekettenreaktion, ist als Kaverne mit einem Boden 102 ausgeführt. Der Boden 102 weist eine Pore 104 auf, durch die das biochemische Material 101, beispielsweise zur Sequenzierung oder Fragmentlängenbestimmung, aus der Analysezelle 100 austreten kann. Die Pore 104 ist als Nanopore ausgebildet. Der Boden 102 ist gemäß diesem Ausführungsbeispiel als Graphenschicht hergestellt, wobei die Pore 104 beim thermischen Umwandeln einer entsprechenden Vorläuferschicht in die Graphenschicht, etwa einer selbstorganisierenden Monoschicht, mithilfe kohlenstoffarmer Vorläufermoleküle mit hydrophober Terminierung erzeugt wurde. Die Vorläuferschicht, die überwiegend aus kohlenstoffreichen Vorläufermolekülen zusammengesetzt ist, wurde dabei in einem geeigneten Beschichtungsverfahren wie beispielsweise Atomlagen-, Gas- oder Flüssigphasenabscheidung mit den kohlenstoffarmen Vorläufermolekülen angereichert. Dabei wurden die kohlenstoffarmen Vorläufermoleküle der Vorläuferschicht in einem definierten Mischungsverhältnis beigemischt, durch das eine mittlere Anzahl von Poren 104 im Boden 102 definiert ist, beispielsweise eine mittlere Anzahl von maximal drei Poren 104. Ferner haben die kohlenstoffarmen Vorläufermoleküle eine definierte Größe, durch die wiederum eine Größe der Pore 104 definiert ist. Durch eine entsprechende Nachbehandlung der Vorläuferschicht, hier durch Erwärmen der Vorläuferschicht, bildet sich an der Einbaustelle zumindest eines der kohlenstoffarmen Vorläufermoleküle eine Lücke in der Kohlenstoffstruktur der beim Erwärmen entstehenden Graphenschicht aus. Diese Lücke stellt die Pore 104 dar.

Die Grundidee des hier beschriebenen Ansatzes besteht darin, in die selbstorganisierende Monoschicht, die als Ausgangsmaterial für die Erzeugung der Graphenschicht auf einer Oberfläche eines entsprechenden Substrats dient, in statistischer Verteilung Moleküle oder Molekülcluster eines zweiten Präkursorentyps einzubauen, die keinen oder nur sehr wenig Kohlenstoff für die Graphenbildung beisteuern. Wird ein solches hinreichend großes Präkursormolekül, auch Vorläufermolekül genannt, zusammen mit anderen Präkursormolekülen, etwa Phenyltrichlorsilan, in die Monoschicht eingebaut, so entsteht bei der nachfolgenden thermischen Behandlung und Konversion der Monoschicht in die Graphenschicht an der Stelle des kohlenstofffreien oder kohlenstoffarmen Moleküls oder Molekülclusters eine Lücke, die dort zur Ausbildung einer Nanopore in der Graphenschicht führt. Dieses kohlenstofffreie Präkursormolekül sollte neben einer hinreichenden Größe auch eine hydrophobe Terminierung aufweisen, beispielsweise durch Stickstoff(N)- bzw. Amid(NH₂)-Gruppen. Die Stickstoffatome oder Amidgruppen erzeugen auch am Ort der speziellen Präkursormoleküle eine hydrophobe Oberfläche. Durch die hydrophobe Terminierung wird an den Stellen eines erfolgten Einbaus dieser Präkursormoleküle ebenfalls eine selbststoppende Eigenschaft erreicht, d. h., es werden auch dort oder darüber keine weiteren kohlenstoffreichen Präkursormoleküle mehr abgeschieden.

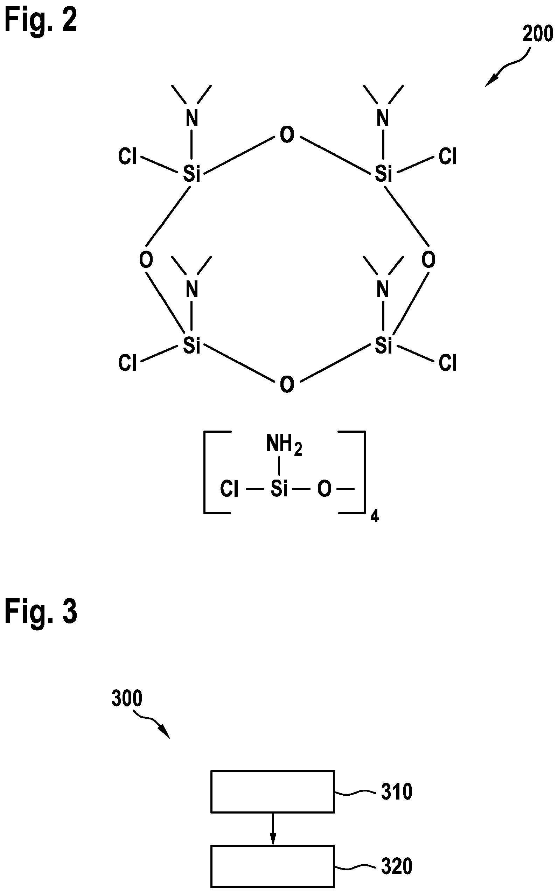

Fig. 2 zeigt eine schematische Darstellung einer Struktur 200 eines kohlenstofffreien Vorläufermoleküls zur Verwendung in einem Verfahren gemäß einem Ausführungsbeispiel. Ein geeignetes Präkursormolekül basiert beispielsweise auf einer Trichlorsilanchemie, wie es in Fig. 2 beispielhaft gezeigt ist. Die Grundstruktur des Präkursormoleküls [Cl-Si(NH₂)-O-]ₐ entspricht bereits einer Basiszelle vom Typ [-O-Si(NH₂)-O-]₄, die sich auf dem Substrat während der Abscheidung periodisch ausbildet. Alternativ können auch andere Moleküle verwendet werden, die sich statistisch in die Monoschicht, beispielsweise aus Phenyltrichlorsilan, einfügen und eine hydrophobe Terminierung der Oberfläche sicherstellen. Es ist auch möglich, von Stickstoff verschiedene kohlenstofffreie oder zumindest kohlenstoffarme Terminierungen zu verwenden, etwa ein einziges Kohlenstoffatom pro Siliziumatom. Im Folgenden wird der Begriff "kohlenstofffrei" gleichgesetzt mit einem Mangel an Kohlenstoff, was auch den Begriff "kohlenstoffarm" mit einschließt.

Die Größe einer "Fehlstelle" bzw. der Nanopore in der später erzeugten Graphenschicht wird bestimmt durch die Größe des speziellen kohlenstofffreien Präkursormoleküls, wobei gemäß einem Ausführungsbeispiel bei der Synthese zur Molekülvergrößerung mehrere Elementarzellen einer Grundstruktur aneinandergesetzt werden, beispielsweise als Molekül der Form [Cl-Si(NH₂)-O-]₄ₙ mit n = 1, 2, 3, ...

Die hier als Beispiel angeführte Verwendung einer Chlorsilanchemie lässt sich sinngemäß auch auf eine Methoxysilanchemie oder Ethoxysilanchemie übertragen, indem Cl durch OCH₃ oder OC₂H₅ ersetzt wird.

Während der thermisch induzierten Umwandlung der Monoschicht erreicht der Kohlenstoff zwar eine gewisse Mobilität, d. h., die Kohlenstoffatome können sich auf der Substratoberfläche zwischen Substratoberseite und Metallunterseite relativ frei bewegen und sich zu einer Graphenschicht reorganisieren. Trotz dieser Beweglichkeit kann der verfügbare Kohlenstoff größere kohlenstofffreie Molekülcluster jedoch nicht überdecken, sodass dort eine Nanopore entsteht, deren Größe oder Durchmesser mit der Größe des speziellen kohlenstofffreien Präkursormoleküls - mit der Ausdehnung des lokalen Kohlenstoffmangels korreliert.

Die Anzahl der Nanoporen korreliert wiederum mit dem relativen Anteil der kohlenstofffreien Präkursormoleküle zu den kohlenstoffreichen Präkursormolekülen, beispielsweise vom Typ Phenyltrichlorsilan. Je mehr kohlenstofffreie Präkursormoleküle beigemischt werden, umso höher ist die Dichte der dadurch erzeugten Nanoporen in der Graphenschicht. Vorteilhaft wird die Dichte der Nanoporen so eingestellt, dass auf die Fläche eines Kavernenbodens der Analysezelle im Mittel jeweils 1 bis 3 Nanoporen, insbesondere 1 bis 2 Nanoporen entfallen. Jeder Boden einer Analysezelle sollte somit mindestens eine und höchstens zwei Nanoporen enthalten. Es ist auch möglich, eine größere Anzahl von Nanoporen pro Analysezelle anzulegen. Dann ist jedoch mit unspezifischen Durchtrittsereignissen zu rechnen, wenn sich gleichzeitig DNA-Fragmente in mehreren Nanoporen bewegen. Diese Situation kann anhand des Stromflussprofils zweifelsfrei erkannt und die betroffenen Datensätze zur Korrektur wieder eliminiert werden. In so einem Fall geht jedoch jedes Mal die Längeninformation der gerade durch die Poren tretenden DNA-Fragmente verloren. Nanoporen außerhalb von Arrayzellen, d. h. Nanoporen, die nicht auf einen Kavernenboden treffen, sind ohne Bedeutung und ohne Funktion. Sie stören nicht den Einsatz der Nanoporen zur Längenbestimmung von DNA-Fragmenten, die aus den qPCR-Zellen durch die Nanoporen am Kavernenboden hindurchtreten. Die Graphenschicht mit den Nanoporen wird je nach Ausführungsbeispiel entweder von einem Trägersubstrat auf einen Arrayzellenwafer kopiert, also transferiert, oder die Abscheidung erfolgt direkt auf dem Arrayzellenwafer unter Einsatz einer zusätzlichen Opferschichttechnik oder einer Stoppschicht für einen DRIE-Prozess (DRIE = deep reactive ion etching; "reaktives lonentiefenätzen"), beispielsweise in Form einer SiO₂-Stoppschicht.

Die Abscheidung der Monoschicht erfolgt beispielsweise aus einer Flüssigphase mit einem Lösungsmittel wie Hexan oder Heptan oder, was besonders vorteilhaft ist, aus einer Gasphase bzw. Dampfphase der Präkursormoleküle. Bei einer Flüssigphasenabscheidung wird dem Lösungsmittel einfach das entsprechende Verhältnis von Phenyltrichlorsilan (oder einem anderen geeigneten SAM-Präkursor) und dem kohlenstofffreien Chlorsilan beigemischt. Bei einer Dampfphasenabscheidung wird das entsprechende Verhältnis über die Partialdrücke der beiden Präkursormoleküle eingestellt, die sich üblicherweise im Hektopascalbereich bewegen. Die kohlenstofffreien Präkursormoleküle sind aus den beschriebenen Gründen deutlich größer und schwerer als beispielsweise Phenyltrichlorsilan und haben daher zu einer gegebenen Temperatur auch einen entsprechend niedrigeren Dampfdruck. Das schadet allerdings nicht, da sie auch nur in deutlich geringerer Konzentration bzw. unter deutlich niedrigerem Partialdruck benötigt werden. Zur Einstellung der Partialdrücke wird die jeweilige Präkursortemperatur und damit der jeweilige Dampfdruck entsprechend gewählt oder es werden Massenflussregler für die Zufuhr der beiden Molekülsorten verwendet. Alternativ werden entsprechende Gasvolumina einmalig oder periodisch aus entsprechend dimensionierten Gasvorratstanks in die eigentliche Reaktionskammer überführt.

Bei einer Atomlagenabscheidung werden zur Erzeugung der Nanoporen neben einem Majoritätspräkursor weitere Minoritätspräkursormoleküle eingesetzt, die beispielsweise eine weitere Abscheidung an Ort und Stelle ihres erfolgten Einbaus unmöglich machen, d. h., beim Wechsel auf den zweiten Majoritätspräkursor werden an den Einbauorten der Minoritätspräkursormoleküle keine neuen Bindungsstellen eröffnet. Nach Abschluss des ALD-Prozesses werden beispielsweise die Minoritätspräkursormoleküle selektiv entfernt, beispielsweise selektiv geätzt, sodass an der betreffenden Stelle eine Nanopore entsteht.

Somit können Graphen- oder ALD-Schichten mit einer gleichmäßigen Verteilung von Nanoporen mit einer vorherbestimmten Größe und Dichte in einem einfachen, schnellen und kostengünstigen Verfahren hergestellt werden. Abgesehen vom Einsatz spezieller Präkursormoleküle sind keine besonderen Anlagen und Einrichtungen erforderlich, die in einer Halbleiter- oder MEMS-Fabrik nicht ohnehin vorhanden sind. Damit ist ein Einsatz des Verfahrens in industriell breit etablierten Strukturen möglich.

In einer Variante erfolgt das Aufwachsen der Monoschicht beispielsweise inselartig bei niedrigen Abscheidetemperaturen von kleiner 20 °C, insbesondere bei 10 °C. Im Folgenden wird ein solches Aufwachsen am Beispiel einer Gasphasenabscheidung beschrieben.

Das Inselwachstum beruht darauf, dass bei niedrigen Temperaturen eine spontane Bekeimung der Substratoberfläche mit statistischer Verteilung durch SAM-Präkursormoleküle stattfindet, die zwar auf der Oberfläche für eine gewisse Zeit mobil sind, insbesondere bei höheren Temperaturen von größer 40 °C, jedoch aufgrund der niedrigen Temperaturen an ihrem Andockort gleichsam eingefroren sind und mit fortschreitender Zeit lateral wachsen. Bei höheren Temperaturen von beispielsweise 70 °C findet hingegen aufgrund der hohen Mobilität der Präkursoren auf der Substratoberfläche eine gleichmäßige Bedeckung mit SAM-Präkursormolekülen statt.

Die Dichte der bei niedriger Temperatur entstehenden Inseln auf der Substratoberfläche ist abhängig von der Reaktivität des Präkursorsystems, dem Partialdruck der Präkursormoleküle, der Abscheidedauer, der Beschaffenheit der Oberfläche selbst und insbesondere von der Dichte der verfügbaren hydrophilen Bekeimungsstellen auf der Oberfläche. Während des Inselwachstums docken weitere Präkursormoleküle an die einmal auf der Oberfläche gebildeten Keime an und vernetzen mit diesen quer durch eine Kondensationsreaktion der Silanolgruppen.

R-Si-(OH)₃+R-Si-(OH)₃→R-Si(OH)₂-O-Si(OH)₂-R+H₂0

Auf diese Weise bauen mehr und mehr Präkursormoleküle ein immer größeres Molekülcluster auf der Oberfläche auf. Nach einer gewissen Zeit, die bei niedrigen Temperaturen von beispielsweise 10 °C mehrere Stunden betragen kann, wird die Vernetzung der Moleküle sowohl untereinander als auch zum Substrat hin irreversibel, d. h., es bauen sich chemische Bindungen der Form -Si-O-Si- sowohl zwischen den Molekülen als auch zu den Bindungsstellen des Substrats hin auf.

Die Dichte der Bekeimungsstellen wird wie folgt gesteuert. Wird ein Silizium- oder Glassubstrat oder eine SiO₂-Oberfläche mit verdünnter Flusssäure HF angeätzt, so verschwinden die hydrophilen OH-Gruppen an der Oberfläche und werden durch eine hydrophobe Terminierung durch H- und F-Atome ersetzt. Diese Oberfläche ist nicht langzeitstabil und wird daher beispielsweise durch Einwirkung von Wasserdampf langsam in eine OH-terminierte Oberfläche zurückverwandelt. Dieser Prozess erfolgt über der Zeitskala statistisch mit einer Gleichverteilung über die Oberfläche. An den rückgebildeten OH-Gruppen docken anschließend SAM-Präkursormoleküle als Keime an, von denen das weitere Inselwachstum ausgeht.

Ein Ausführungsbeispiel des hier vorgestellten Ansatzes besteht nun darin, zunächst in einem ersten Schritt solche Inseln mit kohlenstofffreien oder zumindest kohlenstoffarmen Präkursoren eines ersten Typs zu wachsen. Dies ist beispielsweise mit sehr einfachen Präkursormolekülen möglich, wie etwa HSiCl₃, H₃CSiCl₃, H₃CSi(CH₃O)₃, NH₂Si(CH₃O)₃ oder NH₂Si(C₂H₅O)₃. Es gibt eine Vielzahl solcher kleinen Silane, die nach dem beschriebenen Mechanismus aufwachsen und eine hydrophobe Oberflächenterminierung mit keinem oder nur wenigen Kohlenstoffatomen bereitstellen können. Dazu wird das Wafersubstrat wie vorstehend beschrieben durch Vorbehandlung mit verdünnter Flusssäure HF ganzflächig mit einer hydrophoben Oberfläche versehen. Anschließend wird das Wafersubstrat in eine SAM-Beschichtungsanlage eingeschleust und einer Vorbehandlung mit eingeleitetem Wasserdampf ausgesetzt. Dabei wird über der Zeit kontrolliert eine wachsende Dichte von über die Oberfläche gleichmäßig statistisch verteilten hydrophilen OH-Gruppen erzeugt. Es werden also wenige hydrophile Inseln in statistischer Gleichverteilung über die Oberfläche ausgeformt. Anschließend wird die SAM-Beschichtung bei niedriger Temperatur von 10 bis 20 °C mit den kohlenstofffreien Präkursormolekülen des ersten Typs gestartet, was zu einer Ausbildung kohlenstofffreier oder zumindest kohlenstoffarmer Molekülcluster, den Inseln, auf der Oberfläche führt, die mit der Zeit lateral wachsen.

Sobald genügend Inseln der gewünschten Größe gebildet wurden, wird der erste Schritt des Beschichtungsprozesses unterbrochen und das Wafersubstrat bei niedriger Temperatur gelagert, beispielsweise über 24 Stunden unter Atmosphäre. Während dieser Zeit findet die irreversible Vernetzung der Präkursormoleküle untereinander und mit der Substratoberfläche durch Ausbildung chemischer Bindungen statt. Außerdem werden über diese Zeitspanne durch die Luftfeuchtigkeit oder durch kontrolliert zugeführtem Wasserdampf alle hydrophoben Gruppen (H bzw. F) der Substratoberfläche zu hydrophilen OH-Gruppen umgewandelt. Als Ergebnis entsteht ein hydrophiler Wafer mit hydrophoben kohlenstofffreien Molekülclustern, die gleichmäßig statistisch verteilt und immobilisiert auf der Substratoberfläche vorliegen.

Im nächsten Schritt wird das Wafersubstrat wieder in die SAM-Beschichtungsanlage eingeschleust und mit Wasserdampf erneut konditioniert.

Bei einer erhöhten Temperatur von größer 40 °C, beispielsweise bei 70 °C, findet nun ein zweiter SAM-Beschichtungsschritt mit geeigneten kohlenstoffreichen Präkursormolekülen eines zweiten Typs statt, die nachfolgend durch Hochtemperaturbehandlung unter einer Metallschicht, etwa aus Kupfer oder Nickel, in eine Graphenschicht umgewandelt werden. Dafür wird beispielsweise Octyltrichlorsilan, Decyltrichlorsilan, Phenyltrichlorsilan oder entsprechende Alkoxysilane verwendet. Wesentlich ist, dass diese Schicht nur auf hydrophilen Teilen der Oberfläche aufwächst, wo sich keine kohlenstofffreien Molekülcluster bzw. kohlenstofffreien Inseln befinden, da dort eine hydrophobe Oberfläche vorliegt, über der keine weiteren Präkursormoleküle aufwachsen können. Somit wird eine Substratoberfläche mit einer für die Graphenerzeugung geeigneten Präkursorspezies erzeugt, unterbrochen durch kohlenstofffreie Molekülcluster oder Inseln, die die späteren Nanoporen definieren.

Wie bereits beschrieben, können in die Monoschicht, die als Ausgangsmaterial für die Erzeugung der Graphenschicht auf der Substratoberfläche dient, in statistischer Verteilung Molekülcluster eines abweichenden Präkursorentyps eingebaut werden, wobei die Molekülcluster keinen oder nur sehr wenig Kohlenstoff für die Graphenbildung beisteuern. Die Bildung dieser relativ großen Nanomolekülcluster erfolgt beispielsweise selbstorganisiert aus der Reaktion von kleinen, einfach aufgebauten und leicht verfügbaren kohlenstoffarmen oder kohlenstofffreien Silanen, vorzugsweise in einer Flüssigphase, bevor es zum Kontakt mit der zu beschichtenden Substratoberfläche und zur statistischen Deposition der gebildeten Molekülcluster auf der Substratoberfläche kommt. Liegt ein solches hinreichend großes Präkursormolekülcluster zusammen mit den beispielsweise nachfolgend aufgebrachten Phenyltrichlorsilan-Präkursormolekülen in der Monoschicht auf der Substratoberfläche vor, so entsteht bei der nachfolgenden thermischen Behandlung und Konversion der Monoschicht in die Graphenschicht an der Stelle des kohlenstofffreien Molekülclusters eine Lücke, die dort zur Ausbildung einer Nanopore in der Graphenschicht führt. Dieses kohlenstofffreie Präkursormolekülcluster weist neben einer hinreichenden Größe auch eine hydrophobe Terminierung auf, beispielsweise durch Stickstoff(N)-, Amid(NH₂)-, Methyl-, Kohlenstoff- oder Wasserstoffgruppen. Diese Gruppen erzeugen auch am Ort der speziellen Präkursormolekülcluster eine hydrophobe Oberfläche, wodurch bei einem zweiten Abscheideschritt des Beschichtungsprozesses eine selbststoppende Wirkung eintritt, d. h., es werden auch dort bzw. darüber keine weiteren SAM-Präkursormoleküle mehr abgeschieden.

Gemäß einem weiteren Ausführungsbeispiel wird für eine erste Abscheidung kohlenstofffreier oder kohlenstoffarmer Präkursormolekülcluster auf einer Substratoberfläche eine Flüssigphasenabscheidung eingesetzt. In einer Flüssigphase können Reaktionen der kohlenstofffreien oder kohlenstoffarmen Präkursormoleküle zu Molekülclustern besser als in einer Gasphase kontrolliert werden. Dabei wird die Agglomeration gelöster kohlenstofffreier oder kohlenstoffarmer Präkursormoleküle mit einem Reaktionspartner zu Nanopartikeln oder Nanomolekülclustern und deren nachfolgende Abscheidung auf einer Substratoberfläche kontrolliert ausgenutzt, um Nanoporen in einer später thermisch erzeugten Graphenschicht zu definieren. Ziel ist die Vermeidung einer expliziten Synthese großer Silane. Vielmehr sollen sich auch diese Nanomolekülcluster in einem selbstorganisierenden Prozess aus kleinen, einfach aufgebauten und leicht verfügbaren Silanen bilden und anschließend auf der Substratoberfläche statistisch verteilt abscheiden. Dies wird beispielsweise durch Zusatz einer kleinen Menge Wasser zur Lösung der Präkursormoleküle des ersten Typs in einem geeigneten Lösungsmittel wie Hexan, Heptan oder Chlorkohlen(wasser)stoff initiiert.

Die eigentliche SAM-Beschichtung der gesamten Substratoberfläche mit Ausnahme der bereits vorhandenen kohlenstofffreien oder kohlenstoffarmen Molekülcluster mit einem kohlenstoffreichen Silanpräkursor geschieht hingegen vorzugsweise in einer zweiten Abscheidung mittels Gasphasenabscheidung, um möglichst defektarme und wohldefinierte SAM-Lagen zu erhalten, die nachfolgend eine defektarme Graphenschicht liefern können.

Die Initiierungsreaktion der niedrigen Silane zur Ausbildung von Nanomolekülclustern in Lösung ist eine Hydrolysierung mit dem ebenfalls in geringer Menge zugesetzten Wasser:

R-Si-(Cl)₃+3H₂O→R-Si(OH)₃+3HCl(R=CH₃,NH₂,H,C,...)

Je nach verfügbarem Wassergehalt erfolgt die Hydrolisierung auch nur teilweise, d. h., im letzteren Fall verbleiben sowohl OH- als auch Cl-Gruppen an dem Molekül.

Dadurch wird eine gewisse Menge an im gewählten Beispiel eingesetzten Trichlorsilan zu Hydroxysilan umgesetzt, d. h., es bilden sich abhängig von der Menge an gelöstem Trichlorsilan und vor allem abhängig von der Menge an zugesetztem Wasser Kondensationskeime in der Flüssigphase aus. Die Konzentration der Kondensationskeime hängt von der Menge der Reaktionspartner, vor allem von der Menge an zugesetztem Wasser ab. Nachfolgend und mit fortschreitender Reaktionszeit findet eine Polykondensation der Silanolmoleküle zu Silanolmolekülnanoclustern statt. Dabei können sich sowohl Silanole als auch (unreagierte) Chlorsilane an ein Molekülcluster zusätzlich anlagern und dieses vergrößern. Letztlich wird über den Zeitparameter gesteuert, welche Größe die Nanomolekülcluster erreichen sollen. Nachfolgend sind einige Beispiele für Polykondensationsreaktionen aufgeführt.

R-Si-(OH)₃+R-Si-(OH)₃→R-Si(OH)₂-O-Si(OH)₂-R+H₂O

R-Si-Cl₃+R-Si-(OH)₃→R-Si-(OH)₂-O-SiCl₂-R+HCl

R-Si-Cl3+R-Si(OH)₂-O-Si(OH)₂-R→R-Si(OH)₂-O-Si(OH)-R-O-SiCl₂-R+HCl

R-Si-(OH)₃+R-Si(OH)₂-O-Si(OH)₂-R→R-Si(OH)₂-O-Si(OH)-R-O-Si(OH)₂-R+ H₂O

R-Si-(OH)₃+R-Si(OH)₂-O-SiCl₂-R→R-Si(OH)₂-O-SiCl-R-O-Si(OH)₂-R+HCl

Es gibt also sowohl Kondensationsreaktionen von hydrolisierten oder teilhydrolisierten Molekülen (Silanolen) an ein im Aufbau befindliches Molekülcluster mit Freisetzung von Wasser, was in unmittelbarer Nähe zum im Aufbau befindlichen Molekülcluster weitere Trichlorsilane hydrolisieren kann, oder aber eine direkte Anlagerung von Trichlorsilanen an ein im Aufbau befindliches Molekülcluster. Dichte und Größe von Molekülclustern sind somit zu einem gewissen Grad entkoppelt und unabhängig voneinander kontrollierbar.

Wenn die Größe der Nanomolekülcluster in der Flüssigphase dem gewünschten Wertebereich entspricht, wird das zu beschichtende Substrat mit der Flüssigphase in Kontakt gebracht und die Nanomolekülcluster vermöge ihrer OH-Bindungen oder ihrer Cl-Gruppen an die Substratoberfläche angedockt, wofür hydrophile Bindungsstellen (OH-Gruppen auf der Substratoberfläche) oder sogenannte Bekeimungsstellen besonders attraktiv sind. Als weiterer unterstützender Parameter zur Beeinflussung der Dichte der Nanomolekülcluster, die an die Substratoberfläche binden sollen, kann die Dichte solcher Bekeimungsstellen auf der Substratoberfläche modifiziert werden. Wird ein Siliziumsubstrat oder ein Glassubstrat oder eine SiO₂-Oberfläche mit verdünnter Flusssäure HF angeätzt, so verschwinden die hydrophilen OH-Gruppen an der Oberfläche und werden ersetzt durch eine hydrophobe Terminierung durch H- und F-Atome. Diese Oberfläche ist nicht langzeitstabil und wird beispielsweise durch Einwirkung von Wasserdampf oder an feuchter Atmosphäre langsam in eine OH-terminierte Oberfläche zurückverwandelt. Dieser Prozess erfolgt über der Zeitskala statistisch mit einer Gleichverteilung über die Oberfläche und sehr langsam und ist damit gut über die Zeit kontrollierbar. An den rückgebildeten OH-Gruppen können anschließend die Nanomolekülcluster besonders gut andocken, was neben der Dichte der Nanomolekülcluster in der Flüssigphase einen weiteren Einflussparameter darstellt.

Dazu wird das Wafersubstrat wie vorstehend beschrieben durch Vorbehandlung mit verdünnter Flusssäure HF ganzflächig mit einer hydrophoben Oberfläche versehen. Anschließend wird das Wafersubstrat über eine gewisse Zeit einer wasserdampfhaltigen Atmosphäre ausgesetzt. Dabei wird über der Zeit kontrolliert eine wachsende Dichte von über die Oberfläche gleichmäßig statistisch verteilten hydrophilen OH-Gruppen erzeugt. Dann wird das Wafersubstrat bei vorzugsweise niedriger Temperatur von 10 bis 20 °C in die Flüssigphase eingeführt, in der zuvor aus einem Silanpräkursor des ersten Typs und einer entsprechenden Menge Wasser die gewünschte Dichte und Größe von Silannanomolekülclustern erzeugt wurde. Während der Substratwafer der Flüssigphase ausgesetzt ist, erfolgen ein Andocken von Nanomolekülclustern an die Hydroxylgruppen der Substratoberfläche und eine Ausbildung kohlenstoffarmer Inseln, die aufgrund der niedrigen Temperatur wenig mobil sind und schließlich auf der Substratoberfläche vollständig immobilisieren.

Danach wird das Wafersubstrat bei vorzugsweise niedriger Temperatur von beispielsweise 10 bis 20 °C gelagert, beispielsweise über 24 Stunden unter Atmosphäre. Während dieser Zeit findet die irreversible Vernetzung der Nanomolekülcluster mit der Substratoberfläche durch Ausbildung chemischer Bindungen statt. Außerdem werden über diese Zeitspanne durch die Luftfeuchtigkeit oder durch kontrolliert zugeführtem Wasserdampf alle hydrophoben Gruppen (H oder F) der Substratoberfläche zu hydrophilen OH-Gruppen umgewandelt und alle Chloratome aus der Schicht durch Hydrolyse entfernt. Als Ergebnis entsteht ein hydrophiler Wafer mit hydrophoben kohlenstofffreien Molekülclustern, die gleichmäßig statistisch verteilt und immobilisiert auf der Substratoberfläche vorliegen.

Im nächsten Schritt wird das Wafersubstrat in eine Gasphasen-SAM-Beschichtungsanlage eingeschleust. Bei einer erhöhten Temperatur von größer 40 °C, beispielsweise bei 70 °C, findet nun ein zweiter SAM-Beschichtungsschritt mit geeigneten kohlenstoffreichen Präkursormolekülen eines zweiten Typs statt, die nachfolgend durch Hochtemperaturbehandlung unter einer ebenfalls nachfolgend aufzubringenden Metallschicht, etwa aus Kupfer oder Nickel, in die Graphenschicht umgewandelt werden. Dafür wird beispielsweise Octyltrichlorsilan, Decyltrichlorsilan, Octadecyltrichlorsilan, Phenyltrichlorsilan oder entsprechende Alkoxysilane verwendet. Wesentlich ist, dass diese Schicht nur auf hydrophilen Teilen der Oberfläche aufwächst, wo sich keine kohlenstofffreien Molekülcluster befinden, da dort eine hydrophobe Oberfläche vorliegt, über der keine weiteren Präkursormoleküle aufwachsen können. Im Ergebnis entsteht eine Substratoberfläche mit einer für die Graphenerzeugung geeigneten Präkursorspezies, unterbrochen durch kohlenstofffreie Molekülcluster, die die späteren Nanoporen definieren.

Die Anzahl der Nanoporen korreliert wiederum mit der Anzahl der kohlenstofffreien oder kohlenstoffarmen Präkursormolekülcluster. Je mehr kohlenstofffreie Präkursormolekülcluster auf der Oberfläche vorliegen, umso höher ist die Dichte der dadurch erzeugten Nanoporen in der Graphenschicht. Je größer die kohlenstofffreien Präkursormolekülcluster vor der Abscheidung polykondensiert oder polymerisiert wurden, umso größer wird die jeweilige Nanopore.

Alternativ erfolgt die Erzeugung des Bodens unter Verwendung von Nanopartikeln, die kommerziell erhältlich sind und kostengünstig industriell gefertigt werden. Dadurch können eine einfache Prozessierung und eine gute und einfache Prozesskontrolle erreicht werden.

Dabei werden die Nanopartikel für die erste Abscheidung auf der Substratoberfläche verwendet. Besonders vorteilhaft ist, dass deren Anzahl, Dichte und Größe in einfacher Weise bestimmt werden können und die Unsicherheit einer In-situ-Herstellung aus dem Gesamtprozess eliminiert wird. Der Prozess wird unter wohldefinierten und gut kontrollierten Bedingungen gestartet, was Größe und Anzahl der aufgebrachten Nanopartikel anbelangt.

Zahlreiche Anbieter bieten eine große Auswahl an industriell hergestellten Nanopartikeln in unterschiedlichen Größen ab etwa 10 nm Durchmesser und aus verschiedensten Materialien an, die zu günstigen Preisen in großen Mengen bezogen werden können. Obwohl grundsätzlich eine Vielzahl von Materialien für die vorgeschlagene Methode geeignet ist, erscheint die Verwendung von Silikat-Nanopartikeln aus SiO₂ besonders vorteilhaft. Es existieren zahlreiche Alternativen hinsichtlich der Größe und der Qualität solcher Nanopartikel bis hin zu porösen Nanopartikeln, die mit weiteren Stoffen beladen werden können.

Die zu beschichtende Substratoberfläche wird vorteilhaft zunächst nach einer der bekannten Methoden hydrophilisiert. Danach wird auf die Oberfläche eine Lösung aufgebracht, die die gewünschte Anzahl von Nanopartikeln als Suspension enthält, d. h. eine gewisse Konzentration solcher Nanopartikel dispergiert aufweist. Dabei handelt es sich beispielsweise um die vorangehend erwähnten Silika-Beads. Als Lösungsmittel oder Träger für die Bead-Suspension wird Wasser oder ein organisches Lösungsmittel verwendet, beispielsweise ein Alkohol, Hexan, Heptan oder ein Chlorkohlen(wasser)stoff. Durch Eindampfen des Lösungsmittels verbleibt die vorbestimmte Zahl von Nanopartikeln, vorzugsweise von Silikat-Nanopartikeln, auf der Substratoberfläche. Alternativ zur Aufbringung und Eintrocknung wird die Bead-Dispersion aufgeschleudert, beispielsweise mit einer Waferschleuder. Letzteres ist beispielsweise dann vorteilhaft, wenn es sich bei den Substraten um Siliziumwafer handelt. Bei der Schleudermethode ist die Dichte der Nanopartikel auf der Oberfläche experimentell und in Abhängigkeit von Schleuderdrehzahl und -drehzahlrampe zu ermitteln, da ein Teil der Lösung mit den Nanopartikeln beim Schleudern verloren geht, also noch vor dem Eintrocknen abgeschleudert wird.

Nach dem Trocknen der Substratoberfläche sind die Nanopartikel durch Adhäsionskräfte an die Oberfläche gebunden und werden dort immobilisiert. Bei Verwendung von Silika-Beads gibt es Van-der-Waals-Kräfte über die Hydroxylgruppen, wobei sich durch Kondensationsreaktionen auch bereits erste chemische Bindungen zwischen Substratoberfläche und Nanopartikeln ausbilden können. Die Nanopartikel maskieren somit Teile der Substratoberfläche, auf denen sie immobilisiert sind.

Die auf der Substratoberfläche sitzenden Nanopartikel wirken während des SAM-Beschichtungsprozesses als Maskierung, d. h., da, wo sie sitzen, findet keine Beschichtung der Substratoberfläche statt. Da es sich um annähernd sphärische Nanopartikel handelt, ist die maskierte oder abgedeckte Fläche kleiner als der Durchmesser eines Nanopartikels. Insofern ist ein Durchmesser von 10 bis 20 nm für Silikatnanopartikel durchaus geeignet für eine Maskierung zur Erzeugung von Fehlstehlen, die zu Nanoporen in Graphen führen sollen.

Dabei spielt es keine Rolle, ob die auf der Substratoberfläche sitzenden Nanopartikel selbst mit der Monoschicht beschichtet werden oder nicht. Silikatnanopartikel können über ihre Hydroxylgruppen tatsächlich mit SAM-Präkursoren beschichtet werden. Bei anderen Materialien tritt möglicherweise keine Beschichtung ein.

Nach Abschluss des SAM-Beschichtungsprozesses liegt die Monoschicht auf der Substratoberfläche in chemisch fest gebundener Form vor. Die chemischen Bindungen an die Substratoberfläche über Si-O-Si-Brücken und die Quervernetzung der Silanolgruppen untereinander sind irreversibel.

In diesem Zustand werden die Nanopartikel von der Substratoberfläche entfernt. Dabei werden vorzugsweise mechanische Kräfte eingesetzt, beispielsweise eine Ultraschallreinigung des Wafers in einem Ultraschallbad unterhalb der Kavitationsschwelle oder eine Megasonic-Reinigung des Wafers, d. h. eine Ultraschallreinigung mit Frequenzen im Megahertzbereich, die im Megasonic-Bad keine Kavitation erzeugt und besonders gut zum Entfernen kleinster Teilchen oder Nanopartikel von Substratoberflächen geeignet ist. Möglich ist auch ein Abblasen des Wafers mit Druckluft oder anderen Gasen, ein sanftes Abblasen mit gefrorenem Kohlenstoffdioxid oder gefrorenem Argon (beispielsweise durch Joule-Thomson-Expansion aus einer Düse gefroren) oder ein Abspülen mit einem Wasserstrahl oder anderen bewegten Flüssigkeiten. Die Nanopartikel werden dabei von der Substratoberfläche entfernt, während die Monoschicht dank ihrer stabilen chemischen Bindungen an die Substratoberfläche intakt bleibt. Um die Monoschicht vor Beschädigungen zu schützen, sollten massive Einwirkungen wie etwa Kavitationseffekte unbedingt vermieden werden.

Abgesehen vom Einsatz kommerziell erhältlicher und großtechnisch hergestellter Nanopartikel aus Materialien wie beispielsweise SiO₂, das grundsätzlich halbleiterkompatibel ist, sind keine besonderen Anlagen und Einrichtungen erforderlich, die in einer Halbleiter- oder MEMS-Fabrik nicht ohnehin vorhanden sind. Damit ist ein Einsatz des Verfahrens in industriell breit etablierten Strukturen möglich.

Fig. 3 zeigt ein Ablaufdiagramm eines Verfahrens 300 gemäß einem Ausführungsbeispiel. Das Verfahren 300 zum Herstellen eines Bodens einer Analysezelle, etwa der anhand von Fig. 1 beschriebenen Analysezelle, umfasst einen Schritt 310, in dem das Abscheiden der kohlenstoffreichen und kohlenstoffarmen Vorläufermoleküle, vorangehend auch Präkursormoleküle genannt, auf einem geeigneten Substrat stattfindet. Dabei wird eine Vorläuferschicht gebildet, beispielsweise eine selbstorganisierende Monoschicht als Ausgangsschicht zur Herstellung einer Graphenschicht oder eine ALD-Schicht. In einem weiteren Schritt 320 erfolgt die Nachbehandlung der Vorläuferschicht. Dabei wird die Vorläuferschicht in eine als der Boden fungierende Schicht mit zumindest einer Pore umgewandelt, wobei die Porengröße durch die definierte Größe der kohlenstoffarmen Vorläufermoleküle definiert ist und die Porenanzahl durch das definierte Mischungsverhältnis, in dem die kohlenstoffreichen und kohlenstoffarmen Vorläufermoleküle auf dem Substrat abgeschieden werden, definiert ist.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Verfahren (300) zum Herstellen eines Bodens (102) einer Analysezelle (100) zum Analysieren eines biochemischen Materials (101), wobei das Verfahren (300) folgende Schritte umfasst:
Abscheiden (310) kohlenstoffreicher Vorläufermoleküle und kohlenstoffarmer Vorläufermoleküle in einem definierten Mischungsverhältnis auf einem Substrat, um eine Vorläuferschicht zu bilden, wobei die kohlenstoffarmen Vorläufermoleküle eine definierte Größe und eine hydrophobe Terminierung aufweisen wobei die kohlenstoffreichen Vorläufermoleküle und/oder die kohlenstoffarmen Vorläufermoleküle mittels Atomlagenabscheidung und/oder Gasphasenabscheidung und/oder Flüssigphasenabscheidung abgeschieden werden; und
Nachbehandeln (320) der Vorläuferschicht, um den Boden (102) als Schicht mit zumindest einer Pore (104) mit einer von der definierten Größe abhängigen Porengröße und einer vom definierten Mischungsverhältnis abhängigen Porenanzahl herzustellen.

2. Verfahren (300) gemäß Anspruch 1, bei dem im Schritt des Nachbehandelns (320) die Vorläuferschicht thermisch in eine Graphenschicht mit der Pore (104) umgewandelt wird.

3. Verfahren (300) gemäß einem der vorangegangenen Ansprüche, bei dem im Schritt des Nachbehandelns (320) die kohlenstoffarmen Vorläufermoleküle aus der Vorläuferschicht zumindest teilweise entfernt werden, um die Pore (104) zu erzeugen.

4. Verfahren (300) gemäß einem der vorangegangenen Ansprüche, bei dem im Schritt des Abscheidens (310) die kohlenstoffarmen Vorläufermoleküle in Form von Nanopartikeln abgeschieden werden.

5. Verfahren (300) gemäß einem der vorangegangenen Ansprüche, mit einem Schritt des Bildens von Molekülclustern aus zumindest zwei kohlenstoffarmen Vorläufermolekülen in einer Flüssigphase, wobei im Schritt des Abscheidens (310) die Molekülcluster auf dem Substrat abgeschieden werden, um die Vorläuferschicht zu bilden.

6. Verfahren (300) gemäß Anspruch 5, bei dem im Schritt des Bildens die Molekülcluster durch Polykondensation und/oder Polymerisation gebildet werden.

7. Verfahren (300) gemäß einem der vorangegangenen Ansprüche, bei dem im Schritt des Abscheidens (310) die kohlenstoffreichen Vorläufermoleküle und die kohlenstoffarmen Vorläufermoleküle in einem Mischungsverhältnis abgeschieden werden, das so definiert ist, dass im Schritt des Nachbehandelns (320) der Boden (102) als Schicht mit maximal drei Poren (104) hergestellt wird.

8. Verfahren (300) gemäß einem der vorangegangenen Ansprüche, bei dem im Schritt des Abscheidens (310) eine mit den kohlenstoffarmen Vorläufermolekülen angereicherte selbstorganisierende Monoschicht als die Vorläuferschicht gebildet wird.

9. Verfahren (300) gemäß einem der vorangegangenen Ansprüche, mit einem Schritt des Vorbehandelns des Substrats, um eine hydrophobe Oberfläche des Substrats zu erzeugen, wobei in einem Schritt des Beaufschlagens die hydrophobe Oberfläche mit Wasserdampf beaufschlagt wird, um eine statistisch mit OH-Gruppen angereicherte Oberfläche zu erzeugen, wobei im Schritt des Abscheidens (310) die kohlenstoffarmen Vorläufermoleküle auf der angereicherten Oberfläche abgeschieden werden, um nachfolgend eine hydrophile Oberfläche mit hydrophoben Inseln aus den kohlenstoffarmen Vorläufermolekülen zu erzeugen, wobei die kohlenstoffreichen Vorläufermoleküle auf der nachfolgend erzeugten hydrophilen Oberfläche abgeschieden werden, um die Vorläuferschicht zu bilden.

10. Verfahren (300) gemäß einem der vorangegangenen Ansprüche, bei dem im Schritt des Abscheidens (310) die kohlenstoffarmen Vorläufermoleküle bei einer Temperatur von 5 bis 30 Grad Celsius abgeschieden werden und/oder die kohlenstoffreichen Vorläufermoleküle bei einer Temperatur von größer 40 Grad Celsius abgeschieden werden.

11. Verfahren (300) gemäß einem der vorangegangenen Ansprüche, bei dem im Schritt des Abscheidens (310) die kohlenstoffreichen Vorläufermoleküle nach Abscheiden der kohlenstoffarmen Vorläufermoleküle nach Ablauf einer Vernetzungszeit, während der die abgeschiedenen kohlenstoffarmen Vorläufermoleküle bei einer Vernetzungstemperatur miteinander und/oder mit dem Substrat vernetzt werden, abgeschieden werden.

12. Verfahren (300) gemäß einem der vorangegangenen Ansprüche, bei dem im Schritt des Abscheidens (310) kohlenstoffarme Vorläufermoleküle abgeschieden werden, die durch zumindest eine Stickstoffgruppe und/oder zumindest eine Amidgruppe hydrophob terminiert sind.

## Claims

1. Method (300) for producing a base (102) of an analysis cell (100) for analysis of a biochemical material (101), the method (300) comprising the following steps:
deposition (310) of high-carbon precursor molecules and low-carbon precursor molecules in a defined mixture ratio to a substrate in order to form a precursor layer, wherein the low-carbon precursor molecules have a defined size and a hydrophobic termination, wherein the high-carbon precursor molecules and/or the low-carbon precursor molecules are deposited by means of atomic layer deposition and/or vapour deposition and/or liquid-phase deposition; and
aftertreatment (320) of the precursor layer in order to produce the base (102) as a layer having at least one pore (104) having a pore size dependent on the defined size and a pore number dependent on the defined mixture ratio.

2. Method (300) according to Claim 1, in which in the step of aftertreatment (320), the precursor layer is thermally converted into a graphene layer containing the pore (104).

3. Method (300) according to either of the preceding claims, in which in the step of aftertreatment (320), the low-carbon precursor molecules are at least partially removed from the precursor layer in order to generate the pore (104).

4. Method (300) according to any of the preceding claims, in which in the step of deposition (310), the low-carbon precursor molecules are deposited in the form of nanoparticles.

5. Method (300) according to any of the preceding claims, comprising a step of formation of molecular clusters of at least two low-carbon precursor molecules in a liquid phase, wherein in the step of deposition (310), the molecular clusters are deposited on the substrate in order to form the precursor layer.

6. Method (300) according to Claim 5, in which in the step of formation, the molecular clusters are formed by polycondensation and/or polymerization.

7. Method (300) according to any of the preceding claims, in which in the step of deposition (310), the high-carbon precursor molecules and the low-carbon precursor molecules are deposited in a mixture ratio defined in such a way that in the step of aftertreatment (320), the base (102) is produced as a layer containing not more than three pores (104).

8. Method (300) according to any of the preceding claims, in which in the step of deposition (310), a self-assembled monolayer enriched with the low-carbon precursor molecules is formed as the precursor layer.

9. Method (300) according to any of the preceding claims, comprising a step of pretreatment of the substrate in order to generate a hydrophobic surface of the substrate, wherein in a step of impingement, the hydrophobic surface is impinged upon by water vapour in order to generate a surface randomly enriched with OH groups, wherein in the step of deposition (310), the low-carbon precursor molecules are deposited on the enriched surface in order to subsequently generate a hydrophilic surface containing hydrophobic islands of the low-carbon precursor molecules, wherein the high-carbon precursor molecules are deposited on the subsequently generated hydrophilic surface in order to form the precursor layer.

10. Method (300) according to any of the preceding claims, in which in the step of deposition (310), the low-carbon precursor molecules are deposited at a temperature of 5 to 30 degrees Celsius and/or the high-carbon precursor molecules are deposited at a temperature of greater than 40 degrees Celsius.

11. Method (300) according to any of the preceding claims, in which in the step of deposition (310), the high-carbon precursor molecules are deposited after deposition of the low-carbon precursor molecules upon elapse of a crosslinking time, during which the deposited low-carbon precursor molecules are crosslinked with one another and/or with the substrate at a crosslinking temperature.

12. Method (300) according to any of the preceding claims, in which in the step of deposition (310), low-carbon precursor molecules hydrophobically terminated by at least one nitrogen group and/or at least one amide group are deposited.

## Revendications

1. Procédé (300) de fabrication d'un fond (102) d'une cellule d'analyse (100) destinée à analyser une matière biochimique (101), le procédé (300) comprenant les étapes suivantes :
dépôt (310) de molécules de précurseur riches en carbone et de molécules de précurseur pauvres en carbone dans un rapport de mélange défini sur un substrat, pour former une couche de précurseur, les molécules de précurseur pauvres en carbone présentant une dimension définie et une extrémité hydrophobe, les molécules de précurseur riches en carbone et/ou les molécules de précurseur pauvres en carbone étant déposées par dépôt de couches atomiques et/ou dépôt en phase gazeuse et/ou dépôt en phase liquide ; et
post-traitement (320) de la couche de précurseur, pour réaliser le fond (102) sous forme de couche présentant au moins un pore (104) présentant une dimension de pore dépendant de la dimension définie et un nombre de pores dépendant du rapport de mélange défini.

2. Procédé (300) selon la revendication 1, dans lequel, dans l'étape de post-traitement (320), la couche de précurseur est convertie thermiquement en une couche de graphène présentant le pore (104).

3. Procédé (300) selon l'une des revendications précédentes, dans lequel, dans l'étape de post-traitement (320), les molécules de précurseur pauvres en carbone sont au moins partiellement éliminées de la couche de précurseur pour générer le pore (104).

4. Procédé (300) selon l'une des revendications précédentes, dans lequel, dans l'étape de dépôt (310), les molécules de précurseur pauvres en carbone sont déposées sous forme de nanoparticules.

5. Procédé (300) selon l'une des revendications précédentes, présentant une étape de formation d'agrégats moléculaires constitués par au moins deux molécules de précurseur pauvres en carbone dans une phase liquide, les agrégats moléculaires étant déposés sur le substrat lors de l'étape de dépôt (310) pour former la couche de précurseur.

6. Procédé (300) selon la revendication 5, dans lequel, dans l'étape de formation, les agrégats moléculaires sont formés par polycondensation et/ou polymérisation.

7. Procédé (300) selon l'une des revendications précédentes, dans lequel, dans l'étape de dépôt (310), les molécules de précurseur riches en carbone et les molécules de précurseur pauvres en carbone sont déposées dans un rapport de mélange qui est défini de telle sorte que, dans l'étape de post-traitement (320), le fond (102) est réalisé sous forme de couche présentant au maximum trois pores (104).

8. Procédé (300) selon l'une des revendications précédentes, dans lequel, dans l'étape de dépôt (310), une monocouche auto-organisatrice, enrichie en molécules de précurseur pauvres en carbone, est formée en tant que couche de précurseur.

9. Procédé (300) selon l'une des revendications précédentes, présentant une étape de prétraitement du substrat pour générer une surface hydrophobe du substrat, dans lequel, dans une étape de sollicitation, la surface hydrophobe est soumise à de la vapeur d'eau pour générer une surface enrichie statistiquement en groupes OH, dans lequel, dans l'étape de dépôt (310), les molécules de précurseur pauvres en carbone sont déposées sur la surface enrichie pour générer ensuite une surface hydrophile pourvue d'îlots hydrophobes constitués par les molécules de précurseur pauvres en carbone, les molécules de précurseur riches en carbone étant déposées sur la surface hydrophile générée ensuite pour former la couche de précurseur.

10. Procédé (300) selon l'une des revendications précédentes, dans lequel, dans l'étape de dépôt (310), les molécules de précurseur pauvres en carbone sont déposées à une température de 5 à 30°C et/ou les molécules de précurseur riches en carbone sont déposées à une température supérieure à 40°C.

11. Procédé (300) selon l'une des revendications précédentes, dans lequel, dans l'étape de dépôt (310) les molécules de précurseur riches en carbone sont déposées après le dépôt des molécules de précurseur pauvres en carbone à la fin d'un temps de réticulation, pendant lequel les molécules de précurseur pauvres en carbone déposées sont réticulées les unes avec les autres et/ou avec le substrat à une température de réticulation.

12. Procédé (300) selon l'une des revendications précédentes, dans lequel, dans l'étape de dépôt (310), des molécules de précurseur pauvres en carbone qui sont terminées de manière hydrophobe par au moins un groupe azoté et/ou au moins un groupe amide, sont déposées.
